# EUROPÄISCHE PATENTANMELDUNG

(11) **EP 1 361 535 A1**
(43) Veröffentlichungstag der Anmeldung: **12.11.2003**
(21) Anmeldenummer: 02010468.3
(22) Anmeldetag: 08.05.2002
(51) Int. Cl.: G06F 19/00

(54) **Verfahren und System zur Unterstützung der Therapieplanung**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisermann, Uwe, 91052 Erlangen (DE); Richter, Niels, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Unterstützung der Therapieplanung, insbesondere in der Rehabilitation. Bei dem Verfahren wird ein mehrere Fähigkeitsdefizite umfassendes Fähigkeitsprofil eines Patienten zu Beginn einer Therapie und/oder Information über den Patienten im Rahmen der Therapie verordnete Therapiemodule aus einer Datenbank (1, 1a) bereitgestellt. Jedem der Fähigkeitsdefizite und/oder Therapiemodule werden durch Rückgriff auf eine zweite Datenbank (2, 2a), die für eine Vielzahl von Fähigkeitsdefiziten und/oder Therapiemodulen eine feste Verknüpfung mit therapierenden Organisationseinheiten enthält, automatisch eine oder mehrerer Organisationseinheiten zugeordnet. Zumindest ein Teil der Fähigkeitsdefizite und/oder Therapiemodule wird mit ihrer Zuordnung oder in Abhängigkeit von ihrer Zuordnung an einen Computerarbeitsplatz (10) dargestellt. Das vorliegende Verfahren sowie das zugehörige System vereinfachen die Therapieplanung und Therapieverlaufskontrolle für den behandelnden Arzt oder Therapeuten.

## Beschreibung

Die vorliegende Erfindung betrifft ein Verfahren sowie ein System zur Unterstützung der Therapieplanung, insbesondere in der Rehabilitation.

Als Folge schwerer Erkrankungen wie bspw. Schlaganfall, Herzinfarkt oder Alzheimer'sche Krankheit oder als Folge schwerer operativer Eingriffe wie bspw. dem Einsatz von Gelenkimplantaten oder der Durchführung einer Amputation treten bei der Mehrzahl der Patienten unterschiedliche Defizite in der körperlichen und geistigen Leistungsfähigkeit auf. Diese Defizite sind in der Regel die Folge der Schwächung oder des kompletten Ausfalls einer Gehirnregion oder eines Muskels. Auch Kombinationen hiervon treten häufig auf. So kann z. B. eine Gehirnregion geschädigt sein, die für die Steuerung eines Muskels oder mehrerer Muskeln in funktionalen Ketten zuständig ist. Als Folge davon degenerieren die betroffenen Muskeln, so dass sie nicht mehr richtig eingesetzt werden können. Solche geistigen oder körperlichen Einschränkungen werden in der medizinischen Fachsprache als Fähigkeitsdefizite bezeichnet, die in verschiedene Fähigkeitsbereiche eingeteilt werden können. So ist unterscheidet eine bekannte Klassifizierung bspw.:
- motorische Fähigkeiten wie Kraft, Ausdauer, Beweglichkeit, Gleichgewicht, Reaktion, Orientierung, Differenzierung, Umstellung, Sprachmotorik;
- intellektuelle/kognitive Fähigkeiten wie Aufmerksamkeit, Gedächtnis, Planung, Sprachverständnis, Wortfindung, Sehen;
- organisch/physische Fähigkeiten wie bspw. die Verringerung der Organleistung;
- soziale Fähigkeiten wie bspw. die Kommunikations- und Partizipationsfähigkeit;
- emotionale Fähigkeiten wie bspw. die Fähigkeit zur Entwicklung des Selbstwertgefühls.
Einige Fähigkeiten erfordern auch ein Zusammenspiel motorischer und kognitiver Funktionen. So setzt bspw. die Tätigkeit des Treppensteigens Kraft und Gleichgewicht als motorische Fähigkeiten sowie Aufmerksamkeit und räumliche Wahrnehmung als kognitive Fähigkeiten voraus.

Sehr häufig tritt bei einem Patienten nicht ein einziges Defizit in einer Fähigkeitskategorie auf, sondern eine Kombination mehrerer Defizite in mehr oder weniger schwerer Ausprägung. Ziel einer therapeutischen Maßnahme, die meist im Rahmen eines Rehabilitationsprozesses durchgeführt wird, ist die Wiederherstellung der Fähigkeiten bzw. die weitest mögliche Verminderung der vorliegenden Defizite. Zu Beginn der rehabilitativen Maßnahme werden dabei in der Regel alle Fähigkeitsdefizite des Patienten anhand bekannter Verfahren zur Messung, Beobachtung und Befragung erfasst und ihr Ausmaß dokumentiert. Dieser Erfassungsprozess wird auch als Staging des Patienten bezeichnet. Je nach eingesetztem Messverfahren ist das Ergebnis dieses Staging-Prozesses quantitativ, bspw. in Prozent der Sehfähigkeit oder durch Angabe des Grades der Beweglichkeit des Oberarmes, oder qualitativ, bspw. durch eine Einstufung der Fähigkeitseinschränkung in schwer, mittelgradig oder leicht. Ein Beispiel eines etablierten Messverfahrens für das Staging zahlreicher neurologischer, kognitiver und psychischer Fähigkeiten ist die sog. Wiener Testbatterie der Firma Schuhfried.

Das Resultat dieser Eingangsuntersuchung ist im Idealfall ein fachübergreifender Fähigkeitsbericht, der sich in Form eines Fähigkeitsprofils darstellen lässt. Ein Fähigkeitsprofil wird in diesem Kontext als eine Liste aller relevanten Fähigkeiten und eine Zuordnung des Grades der Einschränkung dieser Fähigkeiten bei diesem Patienten zum Erhebungszeitpunkt definiert.

Neben dem Begriff Fähigkeiten wird in der medizinischen Fachsprache auch der Begriff Fertigkeit benutzt. Unter einer Fertigkeit wird im Kontext einer medizinischen Rehabilitationsmaßnahme eine komplexe Handlung verstanden, die aber in sich geschlossen und gegenüber anderen Handlungen abgrenzbar ist. Für eine Fertigkeit wird das Zusammenspiel mehrerer Fähigkeiten benötigt. Insbesondere bezieht sich der Begriff Fertigkeit im Kontext einer Rehabilitation auf Aktivitäten des täglichen Lebens (ADL: Activities of Daily Living), die Grundvoraussetzung für ein unabhängiges, selbständiges Leben sind. Beispiele für derartige Fertigkeiten sind das Einnehmen von Nahrung, das Anziehen, das Waschen, das Duschen, das Treppensteigen usw.. Die Ausführung solcher Fertigkeiten wird auch in standardisierten Fragebögen erfasst und als ADL-Index quantifiziert. Obwohl im Rahmen einer Rehabilitation in direkter Weise Fähigkeiten trainiert werden, ist das eigentliche Ziel die Wiedererlangung von Fertigkeiten. Insofern sind im Kontext der nachfolgenden Beschreibung die Begriffe Fähigkeit und Fertigkeit meist gegenseitig austauschbar.

In der Regel liegen bei Patienten mehrere Fähigkeitsdefizite gleichzeitig vor, die in den Zuständigkeits- und Kompetenzbereich verschiedener therapierender Organisationseinheiten fallen, wie bspw. unterschiedliche Fachabteilungen einer Rehaklinik oder eines Krankenhauses. Die Abstimmung der Therapieplanung sowie die Kontrolle des Therapiefortschritts zwischen den einzelnen Organisationseinheiten ist dabei eine zeit- und arbeitsaufwendige Aufgabe. Diese Aufgabe muss nicht nur einmalig zu Beginn der Therapie gelöst werden, sondern bedarf einer kontinuierlichen Abstimmung zwischen den einzelnen Organisationseinheiten im gesamten Verlauf des Therapiefortschritts. Bisher erfolgt diese Abstimmung durch zahlreiche Besprechungstermine, mündliche Absprachen und den Austausch von schriftlichen Unterlagen zwischen den beteiligen Organisationseinheiten des Krankenhauses bzw. der Rehaklinik, den betreuenden Ärzten und Therapeuten sowie gegebenenfalls zwischen unterschiedlichen Leistungserbringern einer integrierten Gesundheitsversorgung.

Die Aufgabe der vorliegenden Erfindung besteht darin, ein Verfahren sowie ein System zur Unterstützung der Therapieplanung, insbesondere in der Rehabilitation, anzugeben, das die Therapieplanung hinsichtlich des Zeit- und Arbeitsaufwandes für den einzelnen Arzt oder Therapeuten deutlich vereinfacht.

Die Aufgabe wird mit dem Verfahren sowie dem System gemäß den Patentansprüchen 1 bzw. 13 gelöst. Vorteilhafte Ausgestaltungen des Verfahrens sowie des Systems sind Gegenstand der Unteransprüche oder lassen sich aus der nachfolgenden Beschreibung sowie den Ausführungsbeispielen entnehmen.

Bei dem vorliegenden Verfahren wird ein mehrere Fähigkeitsdefizite umfassendes Fähigkeitsprofil eines Patienten zu Beginn einer Therapie und/oder Information über dem Patienten im Rahmen der Therapie verordnete Therapiemodule aus einer ersten Datenbank bereitgestellt. Jedem der Fähigkeitsdefizite und/oder Therapiemodule werden durch Rückgriff auf eine zweite Datenbank, die für eine Vielzahl von Fähigkeitsdefiziten und/oder Therapiemodulen eine vorgegebene Verknüpfung mit therapierenden Organisationseinheiten enthält, automatisch eine oder mehrere Organisationseinheiten zugeordnet. Anschließend wird zumindest ein Teil der Fähigkeitsdefizite und/oder Therapiemodule mit ihrer Zuordnung oder in Abhängigkeit von ihrer Zuordnung an einem Computerarbeitsplatz dargestellt. Die Darstellung in Abhängigkeit von ihrer Zuordnung bedeutet hierbei, dass die Auswahl der dargestellten Information aufgrund der Zuordnung zu Organisationseinheiten getroffen wird, die Organsiationseinheit selbst jedoch nicht mit dargestellt wird.

Das zugehörige System zur Unterstützung der Therapieplanung umfasst einen Computerarbeitsplatz mit Zugriff auf eine erste Datenbank, die ein mehrere Fähigkeitsdefizite umfassendes Fähigkeitsprofil eines Patienten und/oder Information über dem Patienten verordnete Therapiemodule umfasst, und auf eine zweite Datenbank, die für eine Vielzahl von Fähigkeitsdefiziten und/oder Therapiemodulen eine feste Verknüpfung mit therapierenden Organisationseinheiten enthält. Das System umfasst weiterhin ein Modul, das die Fähigkeitsdefizite und/oder Therapiemodule aus der ersten Datenbank abruft, jedem der Fähigkeitsdefizite und/oder Therapiemodule durch Rückgriff auf die zweite Datenbank automatisch eine oder mehrere Organisationseinheiten zuordnet und zumindest einen Teil der Fähigkeitsdefizite und/oder Therapiemodule mit ihrer Zuordnung oder in Abhängigkeit von ihrer Zuordnung am Computerarbeitsplatz darstellt.

Mit dem vorliegenden Verfahren sowie dem zugehörigen System werden dem Benutzer, insbesondere dem Arzt oder Therapeuten, informationstechnische, computerbasierte Hilfswerkzeuge zur Visualisierung von Patientenstatus und Therapieprozess abteilungsübergreifend bereitgestellt. Der Arzt oder Therapeut ist dadurch in der Lage, am Computerarbeitsplatz eine zeiteffektive und optimal abgestimmte Therapieplanung ohne die Notwendigkeit zahlreicher Besprechungstermine und mündlicher Absprachen mit anderen beteiligten Organisationseinheiten durchzuführen. Durch eine übersichtliche graphische Darstellung aller oder zumindest eines Teils der Fähigkeitsdefizite und/oder Therapiemodule des Patienten in Verbindung mit den zugeordneten therapierenden Organisationseinheiten in den unterschiedlichen Kategorien wird die Therapieplanung erheblich vereinfacht. Die Darstellung der Zuordnung der Fähigkeitsdefizite und/oder Therapiemodule des Patienten zu Organisationseinheiten kann zusätzlich gegliedert nach Organisationskategorien, wie bspw. einzelnen Institutionen des Gesundheitswesens (z.B. Rehaklinik, Krankenhaus, Arztpraxis, physiotherapeutische Praxis), medizinischen Fachdisziplinen oder Fachabteilungen eines Krankenhauses, erfolgen.

In einer Weiterbildung des vorliegenden Verfahrens wird zusätzlich ein aktueller Stand der Fähigkeitsdefizite des Patienten und/oder ein aktueller Leistungsstand des Patienten in den einzelnen Therapiemodulen von der ersten oder einer weiteren Datenbank abgerufen und am Computerarbeitsplatz dargestellt. Auf diese Weise hat der Arzt oder Therapeut jederzeit auch einen Überblick über den aktuellen Stand und die Veränderung der Defizite in nicht zu seinem Fachgebiet gehörigen Bereichen im Verlauf des Behandlungszeitraums. Der aktuelle Leistungsstand des Patienten in den einzelnen Therapiemodulen wird dabei vorzugsweise durch zumindest eine den Therapiefortschritt repräsentierende Maßzahl dargestellt. Eine derartige Maßzahl kann bspw. durch Messung der Leistung des Patienten bei Durchführung einer computergestützten Übung mittels Computerauswertung der Eingaben des Patienten, durch Erfassen von Sensorsignalen an einem Trainingsgerät oder durch Auswertung von Einträgen in medizinischen Patiententagebüchern erhalten werden. Ein Beispiel für eine Erfassung von Maßzahlen ist beispielsweise in der US 6,261,239 angegeben.

In einer weiteren Ausgestaltung des vorliegenden Verfahrens wird den Fähigkeitsdefiziten und/oder Therapiemodulen durch Rückgriff auf eine dritte Datenbank, die für eine Vielzahl von Fähigkeitsdefiziten und/oder Therapiemodulen eine feste Verknüpfung mit zugehöriger Verwaltungsinformation enthält, automatisch Verwaltungsinformation zugeordnet und in Verbindung mit den Fähigkeitsdefiziten und/oder Therapiemodulen am Computerarbeitsplatz dargestellt. Diese Ausgestaltung dient der Vereinfachung von Verwaltungsarbeit, wie bspw. der Abrechnung gegenüber Krankenkassen. Jede einzelne Fähigkeit kann dabei den Verwaltungskriterien wie bspw. den in Deutschland gebräuchlichen Abrechnungscodes ICD oder DRG zugeordnet werden.

Weiterhin ist es bei dem vorliegenden Verfahren möglich, die Zuordnung von Therapiefortschritt, Zuständigkeiten und Verwaltungskriterien nur für ein einzelnes Therapiemodul aus den Datenbanken abzurufen und als graphische Darstellung im Arbeitsablauf am Computerarbeitsplatz verfügbar zu machen.

In einer Weiterbildung des vorliegenden Verfahrens wird zusätzlich eine im Rahmen der Rehabilitation erfasste Compliance des Patienten in den einzelnen Therapiemodulen von der ersten oder einer weiteren Datenbank abgerufen und am Computerarbeitsplatz in Verbindung mit den zugehörigen Therapiemodulen dargestellt. Vorzugsweise wird zum Zwecke einer besseren Übersichtlichkeit in der Darstellung nur diejenige Teilmenge an Information angezeigt, die im jeweiligen Arbeitsschritt relevant ist. So wird bspw. nur die Darstellung und Zuordnung der beteiligten Fachabteilungen angezeigt, wenn es in diesem Arbeitsschritt um die Zeitplanung der Ressourcen innerhalb der Rehaklinik geht. Die Auswahl, welche Teilinformation der Zuständigkeitszuordnung dargestellt wird, kann der Benutzer in einer Ausführung der vorliegenden Erfindung von Hand selbst vornehmen. In der bevorzugten Ausführungsform des vorliegenden Verfahrens sowie des zugehörigen Systems wird diese Auswahl jedoch automatisch kontextabhängig vorgenommen und bei einem Wechsel des Arbeitsschrittes angepasst. Unter der kontextabhängigen Darstellung bzw. Auswahl ist bei dem vorliegenden Verfahren eine Auswahl in Abhängigkeit von dem jeweiligen Arbeitsschritt zu verstehen, den der Benutzer am Computerarbeitsplatz gerade durchführt. Wenn der Benutzer beispielsweise von der Bearbeitung eines Teilschrittes des Therapieplanungsprozesses in einen anderen wechselt, wechselt automatisch die am Bildschirm bereitgestellte Information über Zuständigkeiten.

Bei einer weiteren Ausgestaltung des vorliegenden Verfahrens sowie des zugehörigen Systems wird die für den jeweiligen Arbeitsschritt relevante Information am Bildschirm in Anordnung und graphischer Hervorhebung besonders leicht erkennbar dargestellt. Am Beispiel des Arbeitsschritts der Planung der Klinik-internen Ressourcen wäre dies die Beachtung der Zuständigkeit weiterer Fachabteilungen, die dann in der Darstellung besonders hervor gehoben werden.

Die Darstellung der Fähigkeitsdefizite und/oder Therapiemodule des Patienten sowie ggf. des aktuellen Standes der Fähigkeitsdefizite und/oder des aktuellen Leistungsstandes des Patienten in den Therapiemodulen sowie ggf. weiterer Information erfolgt vorzugsweise in Abhängigkeit von der Organisationseinheit, der der Computerarbeitsplatz zugeordnet ist oder die von einem Benutzer angegeben wird. Bei dieser Ausgestaltung des vorliegenden Verfahrens wird berücksichtigt, dass aus der Gesamtheit der zur Verfügung stehenden Information für Ärzte bzw. Therapeuten unterschiedlicher Fachrichtung unterschiedliche Teilaspekte von Bedeutung sind. In dieser Ausführungsform werden daher nur die für den jeweiligen Benutzer maßgeblichen Informationen dargestellt. Dies kann in Abhängigkeit von der Eingabe des Benutzers erfolgen, in der er seine Fachrichtung bzw. seine Zugehörigkeit zu einer Organisationseinheit oder Kategorie angibt, oder durch entsprechende Vorkonfiguration des Computerarbeitsplatzes in Abhängigkeit von der Organisationseinheit, in der er eingesetzt wird.

In einer bevorzugten Ausführungsform des vorliegenden Verfahrens wird für einen vorgebbaren Zeitraum innerhalb der Therapiedauer eine zeitliche Veränderung des Standes der Fähigkeitsdefizite des Patienten und/oder des Leistungsstandes des Patienten in den Therapiemodulen am Computerarbeitsplatz dargestellt. Auf diese Weise lässt sich eingebettet in den Arbeitsablauf der Therapieplanung und Therapieverlaufskontrolle der aktuelle Stand der Therapie sowie der Verlauf über relevante Zeiträume übersichtlich am Computerarbeitsplatz visualisieren. Neben der Visualisierung des zeitlichen Verlaufs bzw. der zeitlichen Veränderung des Standes der Fähigkeitsdefizite und/oder der Leistung des Patienten können wahlweise auch Differenzen zwischen den jeweiligen Werten zu zwei Zeitpunkten gebildet werden. Bspw. kann die Differenz zwischen aktuellem Stand und Therapiebeginn oder zwischen aktuellem Stand und dem Stand bei der letzten Änderung des verschriebenen Trainings berechnet und am Bildschirm dargestellt werden. Hierbei lassen sich beliebige vom Benutzer vorgebbare Zeiträume als Differenz darstellen. In gleicher Weise können Trenddarstellungen oder Zeitverläufe von mehreren aufeinander folgenden Messzeitpunkten über einen ausgewählten Zeitraum hinweg visualisiert werden. Dem Benutzer steht hierbei eine Vielzahl von Darstellungsmöglichkeiten zur Verfügung, die ihn bei der Therapieplanung und Therapieverlaufskontrolle unterstützen. Selbstverständlich lassen sich die einzelnen Ausgestaltungen des vorliegenden Verfahrens sowie des zugehörigen Systems auch beliebig miteinander kombinieren.

Das vorliegende Verfahren nutzt Datenbanken und graphische Bedienungsoberflächen an einem Computerarbeitsplatz, um im Arbeitsablauf der Therapieplanung und der Therapieverlaufskontrolle zur Behandlung von Fähigkeitsdefiziten einem behandelnden Arzt oder Therapeuten, der in der Regel nur für einen Teil der Fähigkeitsdefizite zuständig ist, schnell und ohne Zusatzaufwand eine übersichtliche Information über den Behandlungsstand weiterer Fähigkeitsdefizite und über weitere am Therapieprozess dieses Patienten beteiligte und zuständige Organisationseinheiten zu geben. In dem Verfahren und dem System zumindest bei einigen der Ausführungsformen eingesetzte Datenbanken, die mit dem Computerarbeitsplatz verbunden sind, umfassen das Fähigkeitsprofil des Patienten zu Beginn der Behandlung, die Fähigkeitsprofile des Patienten zu weiteren Zeitpunkten im Verlauf der Behandlung, die dem Patienten verschriebenen Therapiemodule, den mit einer Maßzahl quantifizierten derzeit erreichten Therapiefortschritt in diesen Therapiemodulen sowie ggf. die quantifizierte Compliance des Patienten in den Therapiemodulen, und die Zuordnung jeder Fähigkeit und jedes Therapiemoduls zur zuständigen Organisationseinheit in verschiedenen Organisationskategorien.

Das vorliegende Verfahren sowie das zugehörige System werden nachfolgend anhand von Ausführungsbeispielen in Verbindung mit den Zeichnungen nochmals kurz erläutert. Hierbei zeigen:
- Fig. 1: ein Beispiel für ein Fähigkeitsprofil eines Patienten;
- Fig. 2: ein Beispiel für die Zuordnung der Fähigkeitsdefizite des Patienten zu therapierenden Organisationseinheiten, gegliedert nach Organisationskategorien;
- Fig. 3: ein Beispiel für die Zuordnung von Fähigkeitsdefiziten des Patienten zu Verwaltungskategorien;
- Fig. 4: ein Beispiel für die Zuordnung von dem Patienten verordneten Therapiemodulen zu Organisationseinheiten, gegliedert nach Organisationskategorien;
- Fig. 5: einen Überblick über ein System zur Unterstützung der Therapieplanung unter Einsatz des vorliegenden Verfahrens;
- Fig. 6: ein Beispiel für die Darstellung des erreichten Leistungsstandes in einzelnen Therapiemodulen im Vergleich zum Leistungsstand bei Behandlungsbeginn;
- Fig. 7: ein Beispiel für die Darstellung des erreichten Standes der Fähigkeitsdefizite im Vergleich zu den Defiziten bei Behandlungsbeginn; und
- Fig. 8: ein Beispiel für die Visualisierung des zeitlichen Verlaufs eines Fähigkeitsdefizites seit Beginn der Behandlung.

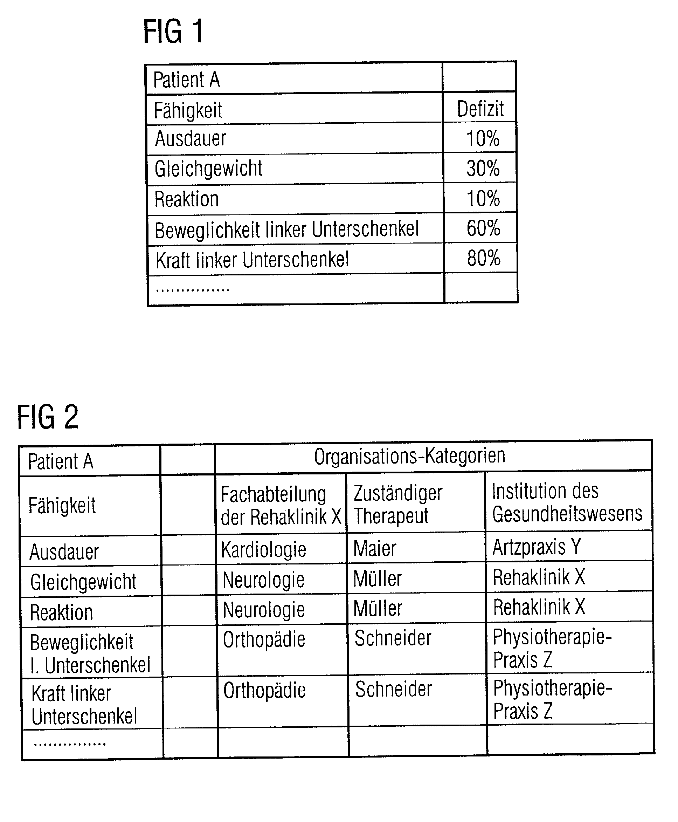

Der Ausgangspunkt des vorliegenden Verfahrens ist ein Computerarbeitsplatz 10 mit Zugriff auf eine Datenbank 1, die das Fähigkeitsprofil eines Patienten einschließlich der Quantifizierung des Grades jedes Defizits nach Abschluss der Eingangsuntersuchungen enthält. Ein Beispiel für ein derartiges Fähigkeitsprofil eines Patienten ist in der Figur 1 dargestellt. Das dort gezeigte Fähigkeitsprofil umfasst die Fähigkeiten Ausdauer, Gleichgewicht, Reaktion, Beweglichkeit des linken Unterschenkels sowie Kraft des linken Unterschenkels.

Das jeweilige Defizit ist in Prozent der entsprechenden 100%igen Fähigkeit einer gesunden Vergleichsperson angegeben.

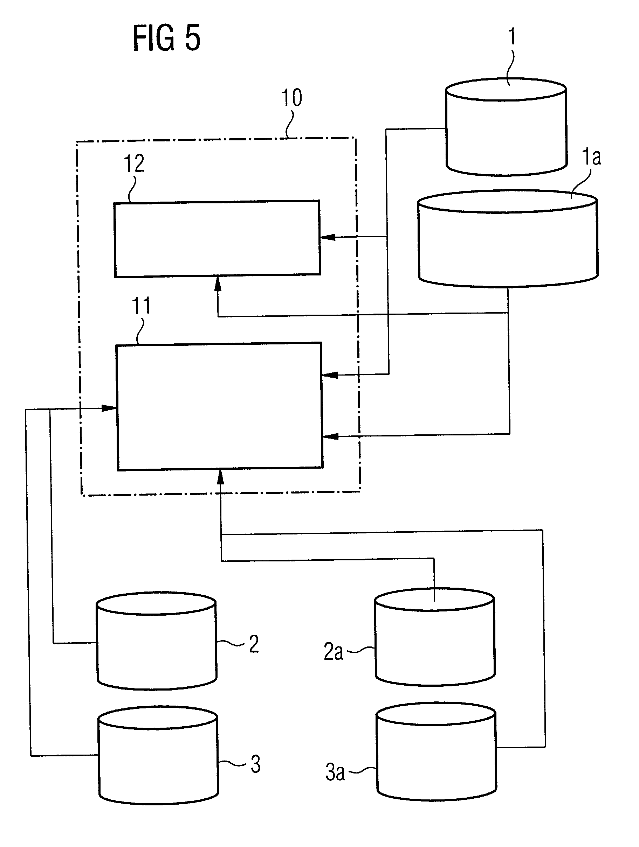

Zusätzlich ist eine zweite Datenbank 2 implementiert, die für eine Vielzahl von Fähigkeiten eine Zuordnung zu therapierenden Organisationseinheiten enthält, so dass durch Zugriff auf die zweite Datenbank 2 jede im Prozess vorkommende Fähigkeit einer zuständigen Organisationseinheit zugeordnet werden kann. Diese Datenbank 2 kann auch eine Zuordnung zu einer oder mehreren Organisationskategorien enthalten. Die Organisationskategorien sind hierbei als Oberbegriff zu Organisationseinheiten zu verstehen. Die entsprechende Vernetzung des Computerarbeitsplatzes 10 mit den beiden Datenbanken 1, 2 ist in der Figur 5 schematisch dargestellt. Der Computerarbeitsplatz 10 enthält hierbei die Module 11, 12, von denen Modul 11 die Fähigkeitsdefizite aus der ersten Datenbank 1 abruft und jedem der Fähigkeitsdefizite durch Rückgriff auf die zweite Datenbank 2 automatisch eine oder mehrere Organisationseinheiten zuordnet und Modul 12 zumindest einen Teil der Fähigkeitsdefizite mit ihrer Zuordnung an einem Monitor des Computerarbeitsplatzes 10 graphisch darstellt.

Figur 2 zeigt ein Beispiel der Zuordnung einzelner Fähigkeitsdefizite zu Organisationseinheiten. Die Organisationseinheiten sind in dieser Darstellung nach Organisationskategorien, insbesondere nach Fachabteilungen der Rehaklinik X, dem zuständigen Therapeuten und der Institution des Gesundheitswesens gegliedert. Aus dieser Darstellung erkennt der behandelnde Arzt oder Therapeut sofort, welche Kollegen anderer Fachrichtungen bei der Rehabilitationsmaßnahme des Patienten zusätzlich involviert sind.

Die Datenbank 2 der Abbildung der einzelnen Fähigkeiten auf dafür zuständige Organisationskategorien bzw. Organisationseinheiten ist spezifisch für den Blickwinkel jeweils einer betrachteten Institution. Somit kann bei der hier vorgeschlagenen Computerarbeitsplatzlösung zur verbesserten Steuerung des Workflows diese Datenbank individuell für jede Organisation implementiert werden.

Da die erste Datenbank 1 mit dem Profil der Fähigkeitsdefizite patientenspezifisch ist, während die zweite Datenbank 2 mit der Abbildung der Fähigkeiten auf die Zuständigkeit verschiedener Organisationskategorien bzw. -einheiten institutionsabhängig ist, bietet sich eine Trennung dieser Datenbanken an. Vertrauliche Patientendaten sollten in einer elektronischen Patientenakte (EPR) gespeichert sein, während organisationsspezifische Daten in einem Hospital Information System (HIS) gespeichert sein können. Selbstverständlich lässt sich letztere Datenbank auch in den Computerarbeitsplatz selbst integrieren. Beide Datenbanken lassen sich jedoch auch als Teildatenbanken einer größeren Datenbank realisieren.

Die dargestellten Datenbanken werden bei dem vorliegenden Verfahren genutzt, um im Arbeitsablauf der Therapieplanung und der Therapieverlaufskontrolle einem Arzt oder Therapeuten, der typischerweise nur für einen Teil der Fähigkeitsdefizite zuständig ist, an einem Computerarbeitsplatz schnell und ohne Zusatzaufwand für diesen, eine übersichtliche Information über weitere am Therapieprozess dieses Patienten beteiligte und zuständige Organisationseinheiten zu geben. Hierfür kann jede beliebige Anordnung und Teilmengeninformation der in den Figuren 1 und 2 dargestellten Listen geeignet sein.

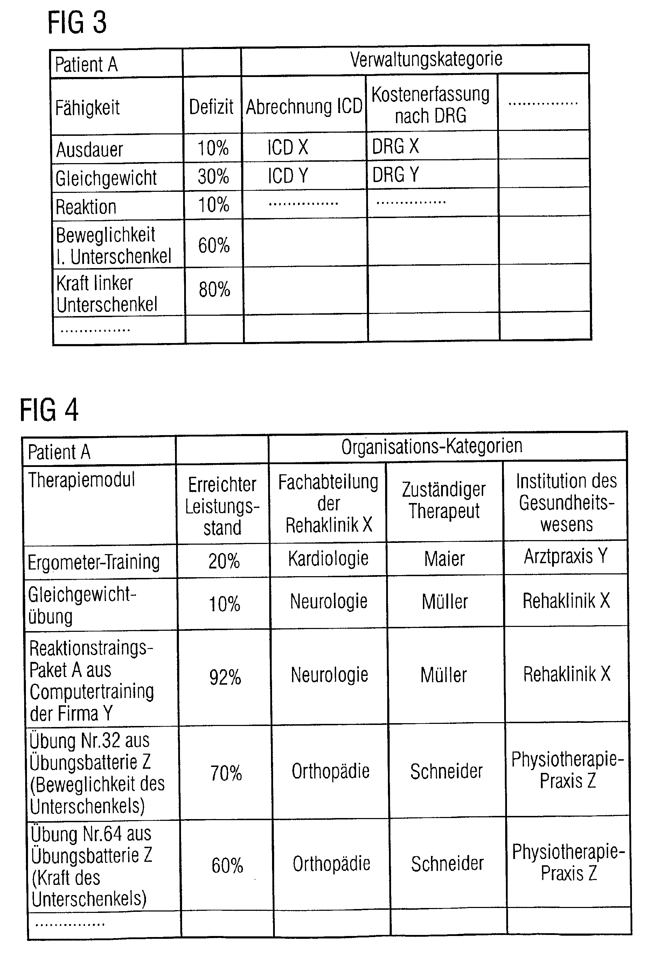

Zusätzlich kann der Computerarbeitsplatz 10 mit einer weiteren Datenbank 3 verbunden sein, die eine Zuordnung einer Vielzahl von Fähigkeiten zu Verwaltungskategorien beinhaltet. Die Module 11, 12 des Computerarbeitsplatzes 10 übernehmen dann eine automatische Zuordnung der Fähigkeiten des Fähigkeitsprofils des Patienten zu den Verwaltungskategorien und stellen diese ebenfalls graphisch dar, wie dies bspw. in Figur 3 zu erkennen ist. Diese Figur zeigt die Fähigkeitsdefizite des Patienten in Zuordnung zu den Verwaltungskategorien Abrechnung nach ICD und Kostenerfassung nach DRG.

Bei dem vorliegenden Verfahren und dem zugehörigen System kann zusätzlich oder an Stelle der Datenbank 1 mit dem individuellen Fähigkeitsprofil auch eine Datenbank 1a mit den dem Patienten verordneten Therapiemodulen und dem zugehörigen Leistungsstand sowie ggf. der Compliance in den einzelnen Therapiemodulen vorgesehen sein. Bei dieser Ausgestaltung wird in gleicher Weise wie bereits in Zusammenhang mit den Fähigkeitsdefiziten erläutert, jedem der Therapiemodule durch das Modul 11 des Computerarbeitsplatzes 10 eine Organisationseinheit durch Rückgriff auf eine Datenbank 2a zugeordnet, die eine entsprechende Zuordnung von Therapiemodulen zu Organisationseinheiten in Organisationskategorien enthält. Ein Beispiel für eine derartige Zuordnung ist in Figur 4 dargestellt, bei der den unterschiedlichen Therapiemodulen, wie bspw. einem Ergometer-Training oder einem Reaktionstrainingspaket, die entsprechenden therapierenden Organisationseinheiten, gegliedert nach Organisationskategorien, zugeordnet sind. In dieser Darstellung ist gleichzeitig der erreichte Leistungsstand in den einzelnen Therapiemodulen als quantitative Maßzahl, hier in Prozent der 100%igen Leistung einer gesunden Vergleichsperson, dargestellt. Auch bei dieser Ausgestaltung lassen sich durch Rückgriff auf eine entsprechende Datenbank 3a, die eine Zuordnung einer Vielzahl von Therapiemodulen zu Verwaltungskategorien enthält, die jeweils dem Patienten verordneten Therapiemodule den entsprechenden Verwaltungskategorien zuordnen und entsprechend darstellen.

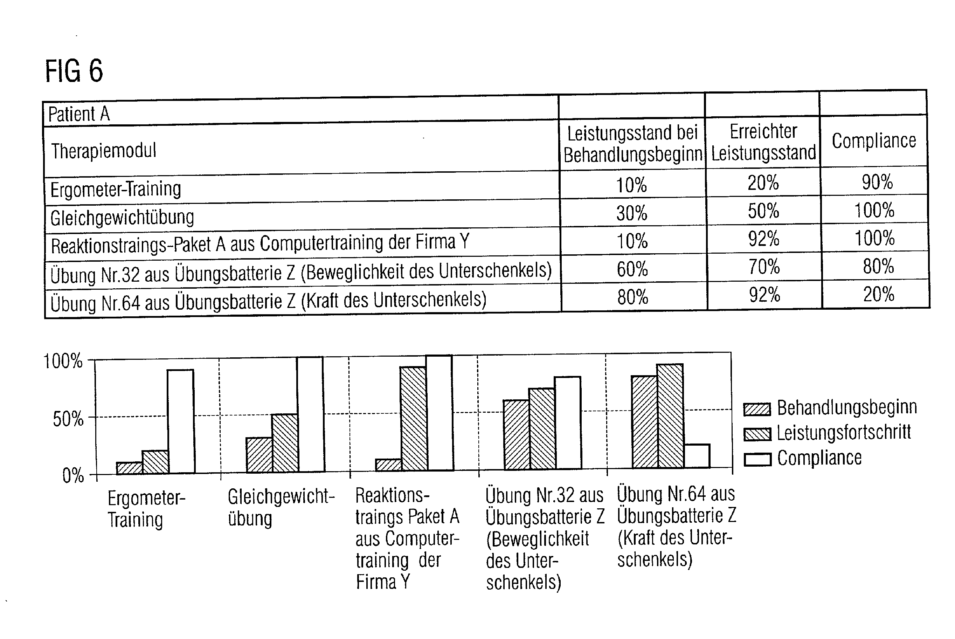

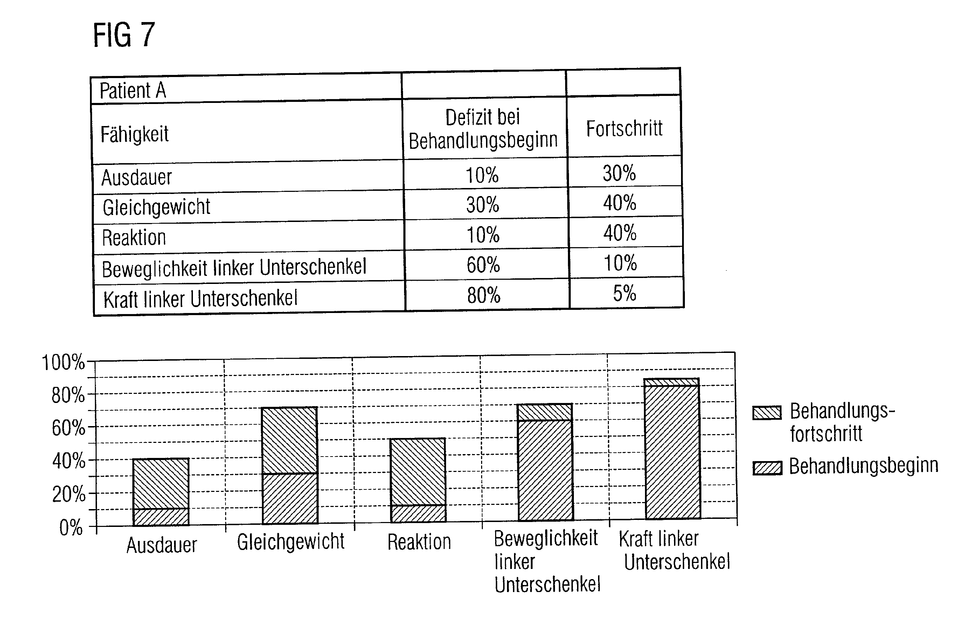

Neben dem jeweils erreichten Leistungsstand kann die Datenbank 1a auch eine quantifizierte Maßzahl für die Compliance des Patienten in dem jeweiligen Therapiemodul enthalten. Diese Information kann für eine Vielzahl unterschiedlicher Darstellungen herangezogen werden. Figur 6 zeigt ein Beispiel einer Darstellung, bei der für die unterschiedlichen dem Patienten verordneten Therapiemodule der Leistungsstand bei Behandlungsbeginn dem momentan erreichen Leistungsstand sowie der Compliance gegenübergestellt werden. In gleicher Weise können die zur Verfügung stehenden Datenbanken der Fähigkeitsprofile und Therapiemodule mit quantifiziertem Therapiefortschritt bzw. Compliance genutzt werden, um in übersichtlicher Art und Weise den aktuellen Stand der Therapie und den Verlauf über relevante Zeiträume am Computerarbeitsplatz zu visualisieren. Die quantifizierten Werte für Fähigkeitsdefizit, Leistungsstand bei einer Übung oder Compliance können bspw. als Balkendiagramm oder in Farbskalierung dargestellt werden. Von besonderem Vorteil ist die durch die gemeinsame Datenbank aller Fähigkeitsdefizite bzw. aller Therapiemodule ermöglichte Übersicht über das Gesamtbild des Behandlungsprozesses. Figur 7 zeigt hierzu ein weiteres Beispiel, bei dem die Fähigkeitsdefizite zu Behandlungsbeginn dem bisher erreichten Fortschritt gegenübergestellt werden.

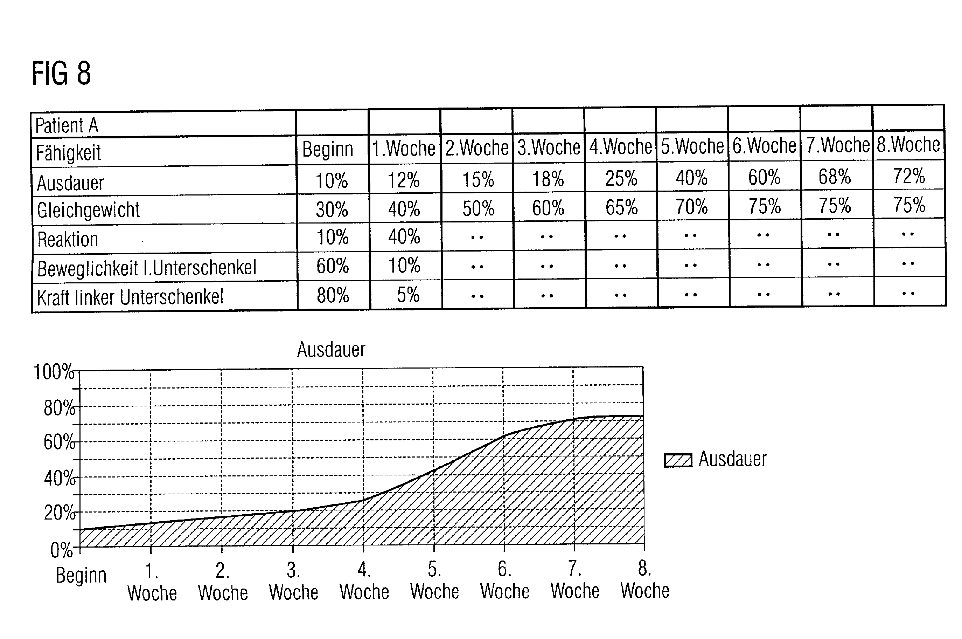

Figur 8 zeigt ein Beispiel für eine weitere Darstellungsmöglichkeit beim vorliegenden Verfahren sowie dem zugehörigen System. Bei diesem Ausführungsbeispiel wird der zeitliche Verlauf eines Fähigkeitsdefizits, im vorliegenden Falle der Ausdauer, vom Beginn der Therapie bis zum momentanen Erfassungszeitpunkt visualisiert. Selbstverständlich ist es möglich, auch andere Fähigkeitsdefizite, den Leistungsstand in einzelnen Therapiemodulen sowie die zugehörige Compliance in einer vergleichbaren Weise zu visualisieren.

## Patentansprüche

1. Verfahren zur Unterstützung der Therapieplanung, insbesondere in der Rehabilitation, bei dem
- ein mehrere Fähigkeitsdefizite umfassendes Fähigkeitsprofil eines Patienten zu Beginn einer Therapie und/oder Information über dem Patienten im Rahmen der Therapie verordnete Therapiemodule aus einer Datenbank (1, 1a) bereitgestellt wird,
- jedem der Fähigkeitsdefizite und/oder Therapiemodule durch Rückgriff auf eine zweite Datenbank (2, 2a), die für eine Vielzahl von Fähigkeitsdefiziten und/oder Therapiemodulen eine feste Verknüpfung mit therapierenden Organisationseinheiten enthält, automatisch eine oder mehrere Organisationseinheiten zugeordnet werden, und
- zumindest ein Teil der Fähigkeitsdefizite und/oder Therapiemodule mit ihrer Zuordnung oder in Abhängigkeit von ihrer Zuordnung an einem Computerarbeitsplatz (10) dargestellt wird.

2. Verfahren nach Anspruch 1,
**dadurch gekennzeichnet,**
**dass** die Zuordnung und Darstellung der Zuordnung der Fähigkeitsdefizite und/oder Therapiemodule des Patienten zu Organisationseinheiten gegliedert nach übergeordneten Organisationskategorien erfolgt.

3. Verfahren nach Anspruch 1 oder 2,
**dadurch gekennzeichnet,**
**dass** ein aktueller Stand der Fähigkeitsdefizite des Patienten und/oder ein aktueller Leistungsstand des Patienten in den Therapiemodulen von der ersten (1, 1a) oder einer weiteren Datenbank abgerufen und am Computerarbeitsplatz (10) dargestellt wird.

4. Verfahren nach Anspruch 3,
**dadurch gekennzeichnet,**
**dass** der aktuelle Leistungsstand des Patienten in den Therapiemodulen durch zumindest eine den Therapiefortschritt repräsentierende Maßzahl dargestellt wird.

5. Verfahren nach einem der Ansprüche 1 bis 4,
**dadurch gekennzeichnet,**
**dass** den Fähigkeitsdefiziten und/oder Therapiemodulen durch Rückgriff auf eine dritte Datenbank (3, 3a), die für eine Vielzahl von Fähigkeitsdefiziten und/oder Therapiemodulen eine feste Verknüpfung mit zugehöriger Verwaltungsinformation enthält, automatisch Verwaltungsinformation zugeordnet und wahlweise in Verbindung mit den Fähigkeitsdefiziten und/oder Therapiemodulen am Computerarbeitsplatz (10) dargestellt wird.

6. Verfahren nach einem der Ansprüche 1 bis 5,
**dadurch gekennzeichnet,**
**dass** eine erfasste Compliance des Patienten in den Therapiemodulen von der ersten (1, 1a) oder einer weiteren Datenbank abgerufen und am Computerarbeitsplatz (10) in Verbindung mit den Therapiemodulen dargestellt wird.

7. Verfahren nach einem der Ansprüche 1 bis 6,
**dadurch gekennzeichnet,**
**dass** die Darstellung der Fähigkeitsdefizite und/oder Therapiemodule des Patienten sowie gegebenenfalls des aktuellen Standes der Fähigkeitsdefizite und/oder des aktuellen Leistungsstandes des Patienten in den Therapiemodulen und gegebenenfalls weiterer Informationen kontextabhängig oder in Abhängigkeit von der Auswahl eines Benutzers erfolgt.

8. Verfahren nach einem der Ansprüche 1 bis 7,
**dadurch gekennzeichnet,**
**dass** für den jeweiligen Arbeitsschritt relevante Information in der Darstellung graphisch hervorgehoben wird.

9. Verfahren nach einem der Ansprüche 1 bis 8,
**dadurch gekennzeichnet,**
**dass** die Darstellung der Fähigkeitsdefizite und/oder Therapiemodule des Patienten sowie gegebenenfalls des aktuellen Standes der Fähigkeitsdefizite und/oder des aktuellen Leistungsstandes des Patienten in den Therapiemodulen und gegebenenfalls weiterer Informationen in Abhängigkeit von der Organisationseinheit erfolgt, der der Computerarbeitsplatz (10) zugeordnet ist oder die von einem Benutzer angegeben wird.

10. Verfahren nach einem der Ansprüche 1 bis 6,
**dadurch gekennzeichnet,**
**dass** in Abhängigkeit von der Eingabe eines Benutzers nur ein einzelnes Therapiemodul mit der oder den zugeordneten Organisationseinheiten und/oder dem Therapiefortschritt und/oder der Verwaltungsinformation und/oder der Compliance dargestellt wird.

11. Verfahren nach einem der Ansprüche 1 bis 10,
**dadurch gekennzeichnet,**
**dass** für einen vorgebbaren Zeitraum innerhalb der Therapiedauer eine zeitliche Veränderung des Standes der Fähigkeitsdefizite des Patienten und/oder des Leistungsstandes des Patienten in den Therapiemodulen am Computerarbeitsplatz (10) dargestellt wird.

12. Verfahren nach einem der Ansprüche 1 bis 11,
**dadurch gekennzeichnet,**
**dass** nach Vorgabe von zwei Zeitpunkten innerhalb der Therapiedauer eine Differenz im Stand der Fähigkeitsdefizite des Patienten und/oder im Leistungsstand des Patienten in den Therapiemodulen und/oder in der Compliance zwischen den beiden Zeitpunkten errechnet und in Verbindung mit den Fähigkeitsdefiziten und/oder Therapiemodulen am Computerarbeitsplatz (10) dargestellt wird.

13. System zur Unterstützung der Therapieplanung, insbesondere in der Rehabilitation, bestehend aus
- einem Computerarbeitsplatz (10) mit Zugriff auf eine erste Datenbank (1, 1a), die mehrere Fähigkeitsdefizite eines Patienten und/oder Information über dem Patienten verordnete Therapiemodule umfasst, und auf eine zweite Datenbank (2, 2a), die für eine Vielzahl von Fähigkeitsdefiziten und/oder Therapiemodulen eine feste Verknüpfung mit therapierenden Organisationseinheiten enthält, und
- einem Modul (11, 12), das die Fähigkeitsdefizite und/oder Therapiemodule aus der ersten Datenbank (1, 1a) abruft, jedem der Fähigkeitsdefizite und/oder Therapiemodule durch Rückgriff auf die zweite Datenbank (2, 2a) automatisch eine oder mehrere Organisationseinheiten zuordnet und zumindest einen Teil der Fähigkeitsdefizite und/oder Therapiemodule mit ihrer Zuordnung oder in Abhängigkeit von ihrer Zuordnung am Computerarbeitsplatz (10) darstellt.

14. System nach Anspruch 13,
**dadurch gekennzeichnet,**
**dass** die erste (1, 1a) und/oder zweite Datenbank (2, 2a) in den Computerarbeitsplatz (10) integriert sind.

15. System nach Anspruch 13,
**dadurch gekennzeichnet,**
**dass** die erste (1, 1a) und/oder zweite Datenbank (2, 2a) über ein Netzwerk mit dem Computerarbeitsplatz (10) verbunden sind.

16. System nach einem der Ansprüche 13 bis 15,
**dadurch gekennzeichnet,**
**dass** die erste (1, 1a) oder eine weitere Datenbank, mit der der Computerarbeitsplatz (10) verbunden ist, einen aktuellen Stand der Fähigkeitsdefizite des Patienten und/oder einen aktuellen Leistungsstand des Patienten in den Therapiemodulen enthält, und das Modul (11, 12) derart ausgebildet ist, dass es den aktuellen Stand der Fähigkeitsdefizite des Patienten und/oder den aktuellen Leistungsstand des Patienten in den Therapiemodulen automatisch oder nach einer Eingabe eines Benutzers abruft und am Computerarbeitsplatz (10) dargestellt.

17. System nach einem der Ansprüche 13 bis 16,
**dadurch gekennzeichnet,**
**dass** die zweite (2, 2a) oder eine dritte Datenbank (3, 3a), die mit dem Computerarbeitsplatz (10) verbunden ist, für eine Vielzahl von Fähigkeitsdefiziten und/oder Therapiemodulen eine feste Verknüpfung mit zugehöriger Verwaltungsinformation enthält, und das Modul (11, 12) derart ausgebildet ist, dass es die Verwaltungsinformation automatisch oder nach einer Eingabe eines Benutzers abruft und in Verbindung mit den Fähigkeitsdefiziten und/oder Therapiemodulen am Computerarbeitsplatz (10) darstellt.

18. System nach einem der Ansprüche 13 bis 17,
**dadurch gekennzeichnet,**
**dass** das Modul (11, 12) derart ausgebildet ist, dass es nach einer Eingabe eines Benutzers eine zeitliche Veränderung des Standes der Fähigkeitsdefizite des Patienten und/oder des Leistungsstandes des Patienten in den Therapiemodulen für einen vorgebbaren Zeitraum durch Rückgriff auf die erste Datenbank (1, 1a) ermittelt und am Computerarbeitsplatz (10) darstellt.
